# EUROPEAN PATENT SPECIFICATION

(11) **EP 3 393 366 B1**
(45) Date of publication and mention of the grant of the patent: **12.02.2025**
(21) Application number: 16816666.8
(22) Date of filing: 19.12.2016
(51) Int. Cl.: A61B 8/00, A61B 8/08, G16H 50/30

(54) **ULTRASOUND IMAGING APPARATUS AND ULTRASOUND IMAGING METHOD FOR INSPECTING A VOLUME OF SUBJECT**
ULTRASCHALLBILDGEBUNGSVORRICHTUNG UND ULTRASCHALLBILDGEBUNGSVERFAHREN ZUR INSPEKTION EINES VOLUMENS EINER PERSON
APPAREIL D'IMAGERIE ULTRASONORE ET PROCÉDÉ D'IMAGERIE ULTRASONORE POUR L'INSPECTION D'UN VOLUME DE SUJET

(30) Priority: 21.12.2015 EP 15307070
(43) Date of publication of application: 31.10.2018
(73) Proprietor: Koninklijke Philips N.V., 5656 AG Eindhoven (NL)
(72) Inventor: ROUET, Laurence, 5656 AE Eindhoven (NL); COLLET-BILLON, Antoine, 5656 AE Eindhoven (NL); ENTREKIN, Robert Randall, 5656 AE Eindhoven (NL)
(74) Representative: Philips Intellectual Property & Standards
(86) International application number: PCT/EP2016/081684
(87) International publication number: WO 2017/108667

(56) References cited:
- EP-A1- 1 685 799
- EP-A1- 2 612 599
- EP-A1- 2 807 978
- US-A1- 2012 071 758
- US-A1- 2014 343 428
- US-A1- 2015 359 512

## Description

### FIELD OF THE INVENTION

The present invention relates to an ultrasound imaging apparatus for inspecting a volume of a subject. The present invention further relates to an ultrasound imaging method for inspecting a volume of a subject. The present invention in particular relates to real-time user guidance and optimization of image quality during ultrasound inspection of a volume of a subject in order to provide a size measurement of an anatomical object of the subject based on ultrasound data.

### BACKGROUND OF THE INVENTION

In the field of medical imaging systems three-dimensional ultrasound measurement units are well-known for quantification analysis and size measurements such as diameters or volumes of anatomical objects of a patient. A corresponding ultrasound apparatus is e.g. known from WO 2014/097090 A1.

The three-dimensional ultrasound systems have due to the huge amount of measurement data in the field of view a reduced spatial resolution and a reduced image quality in real-time so that the three-dimensional ultrasound systems have a limited usability for precise quantification analysis or size measurements. Further, since the usability of ultrasound measurements for quantification analysis or size measurements is highly dependent on the image quality of the ultrasound data and since the image quality is highly dependent on the viewing direction of the ultrasound probe and the direction of the ultrasound beams with respect to the anatomical structures to be measured, extensive user experiences are necessary to achieve high quality ultrasound images for a precise ultrasound quantification or size measurements.

EP 2 612 599 A1 discloses a method and device for evaluating the quality of elasticity volume data in order to improve the quality of a three-dimensional elasticity image.

US 2014/343428 A1 discloses an ultrasound diagnosis apparatus comprising, a setting unit, a virtual endoscopic image generating unit and a display controlling unit. The setting unit configured to, on a basis of information about a lumen rendered in a two-dimensional tomographic image generated by using three-dimensional image data, set a value of an image quality adjusting parameter used for generating a virtual endoscopic image obtained by projecting an inside of the lumen from a predetermined viewpoint.

US 2015/359512 A1 discloses a synthetic aperture ultrasound system includes an ultrasound probe, and an ultrasound signal processor configured to communicate with the ultrasound probe to receive phase and amplitude information from a plurality of ultrasonic echo signals from a corresponding plurality of ultrasound pulses.

EP 1 685 799 A1 discloses an ultrasonic diagnostic apparatus capable of acquiring a three-dimensional image, and more particularly to an ultrasonic diagnostic apparatus for extracting a three-dimensional image of a region of interest.

EP 2 807 978 A1 discloses methods and systems used in ultrasound (US) imaging of biological soft tissues. More specifically, it relates to an US-acquisition protocol with an interactive real-time feedback to the user.

US 2012/071758 A1 discloses techniques and supporting systems that facilitate real-time or near-real-time ultrasound tracking for the purpose of calculating changes in anatomical features during a medical procedure.

### SUMMARY OF THE INVENTION

It is therefore an object of the present invention to provide an improved ultrasound imaging apparatus which provides an improved image quality with low technical effort. It is a further object to provide a corresponding ultrasound imaging method.

According to one aspect of the present invention, an ultrasound imaging apparatus for inspecting a volume of a subject according to claim 1 is disclosed.

According to another aspect of the present invention, an ultrasound imaging method for inspecting a volume of a subjec according to claim 12 is disclosed.

Preferred embodiments of the invention are defined in the dependent claims. It shall be understood that the claimed method has similar and/or identical preferred embodiments as the claimed device and as defined in the dependent claims.

The present invention is based on the idea to acquire ultrasound data in a field of view by means of an ultrasound probe and to provide two-dimensional ultrasound image data in two different image planes on the basis of the ultrasound data and to determine a quality parameter on the basis of the positional relation of an anatomical feature with respect to the image planes of the two-dimensional ultrasound image data in the field of view. The two-dimensional ultrasound image data in the field of view is provided as real-time image data in bi-planar mode in the two different image planes. On the basis of the so-determined quality parameter and the positional relation of the anatomical feature with respect to the two image planes or the field of view or a depth of the two-dimensional ultrasound image data, the image planes or the field of view can be adapted in order to improve the viewing direction of the ultrasound probe so that ultrasound images can be provided with the highest quality for quantification purposes or for size measurements. Since the determination of the anatomical feature is based on the two-dimensional ultrasound image data, the image quality of the ultrasound image data can be improved, in particular in real-time, and the image planes can be aligned or an alignment can be indicated. Hence, 3D ultrasound image data can be acquired based on the aligned probe position having an improved image quality and a precise quantification and / or measurement of anatomical features can be achieved.

In a preferred embodiment the ultrasound probe is adapted to acquire the two-dimensional ultrasound image data in the two different image planes in the field of view simultaneously. This is a possibility to provide different two-dimensional image data in real-time and to guide the user precisely in real-time to cover the region of interest and to achieve high quality image data.

In a further preferred embodiment, the two different image planes are disposed perpendicular to each other. The ultrasound probe is in particular adapted to use a bi-planar mode in order to provide different two-dimensional images in different image planes. This is a possibility to determine the two-dimensional ultrasound image data in a larger field of view so that the ultrasound data acquisition and the alignment of the ultrasound probe with respect to the anatomical feature can be improved.

In a preferred embodiment, the ultrasound imaging apparatus further comprises a display unit for displaying an ultrasound image on the basis of the two-dimensional ultrasound image data. This is a possibility to align the image plane of the ultrasound probe on the basis of a visual inspection of the ultrasound image by a user.

In a preferred embodiment, the alignment unit is adapted to indicate a movement direction of the ultrasound probe on a display unit based on the positional relation and the quality parameter. This is a possibility to provide a user guidance as a feedback so that the user can perform a manual alignment of the ultrasound probe in order to improve the quality of the ultrasound image data by adapting the positional relation between the anatomical feature and the ultrasound probe.

In a preferred embodiment, the alignment unit is adapted to indicate the quality parameter with respect to predefined quality limits on the display unit. This is a possibility to quantify the positional relation and the quality of the image data with low technical effort.

In a preferred embodiment, the movement direction is indicated within the ultrasound image. This is a possibility to provide a simple feedback and a comfortable user guidance for manual alignment of the ultrasound probe.

In a preferred embodiment, the ultrasound imaging apparatus further comprises a control unit for controlling a steering direction of the ultrasound probe based on the positional relation and the quality parameter. This is a possibility to provide an automatic alignment of the image plane based on the positional relation and the quality parameter so that the best image quality can be provided automatically without a manual alignment by the user.

In a preferred embodiment, the positional relation is a distance of a center of the anatomical feature from a center of the two-dimensional ultrasound image in the image plane. This is a possibility to determine the position of the ultrasound probe with respect to the anatomical feature with low technical effort on the basis of the two-dimensional ultrasound image data.

In a further preferred embodiment, the positional relation is an angle between a longitudinal axis of the anatomical feature with respect to a horizontal axis of the two-dimensional ultrasound image in the image plane. This is a possibility to determine an alignment of the anatomical feature with respect to a propagation direction of the ultrasound waves emitted by the ultrasound probe so that the quality parameter can be determined with low technical effort and high precision.

In a further preferred embodiment, the positional relation is an outline of the anatomical feature with respect to the field of view in the image plane. The positional relation is in particular a size of an outline of the anatomical feature with respect to a size of the field of view in the image plane. This is a possibility to determine the quality parameter based on the positional relation of the anatomical feature with respect to the borders of the two-dimensional ultrasound image and to determine whether the anatomical feature is displayed within or partially outside the two-dimensional ultrasound image.

According to a preferred embodiment, the positional relation is a position of the anatomical feature with respect to an image depth of the ultrasound image data. This is a possibility to determine whether the image depth of the ultrasound image data is adapted to the position of the anatomical feature in the beaming direction of the ultrasound waves or whether the image depth is too large or too small.

According to a preferred embodiment, the image processing unit is adapted to receive the two-dimensional ultrasound image data as a continuous data stream and to determine the quality parameter based on the positional relation of the anatomical feature with respect to the image plane in real time. This is a possibility to continuously align the anatomical feature to the ultrasound probe so that a quantification and/or a size measurement can be performed comfortable with low time consumption.

In a preferred embodiment, the image processing unit comprises a measurement unit for measuring a size of the anatomical feature on the basis of the two-dimensional image data. This is a possibility to further reduce the time consumption for the measurement of the anatomical feature, since the measuring process is performed based on the two-dimensional image data.

In a further preferred embodiment, not part of the present invention, the image processing unit comprises a measurement unit for measuring a size of the anatomical feature on the basis of the three-dimensional ultrasound image data. This is a possibility to determine the size of the anatomical feature with high precision based on the three-dimensional ultrasound image data.

In a preferred embodiment, not part of the present invention, the measurement unit comprises a segmentation unit for providing segmentation data of the three-dimensional ultrasound image data or the two-dimensional ultrasound image data and for measuring a size of the anatomical feature on the basis of the segmentation data. This is a possibility to measure the size of the anatomical feature with high precision based on the three-dimensional ultrasound image data or the two-dimensional ultrasound image data.

In a preferred embodiment, the evaluation unit comprises a segmentation unit for providing segmentation data of the anatomical feature in the two-dimensional ultrasound image data and for determining the positional relation on the basis of the segmentation data. This is a possibility to precisely determine the positional relation of the anatomical feature with respect to the image plane and the ultrasound probe and to precisely determine the quality parameter.

In a preferred embodiment, the evaluation unit is adapted to determine the quality parameter based on a positional relation of the anatomical feature with respect to the two different image planes. This is a possibility to improve the measurement of the anatomical feature, since the alignment of the anatomical feature with respect to the ultrasound probe is based on two different image planes.

In a preferred embodiment, the anatomical feature is a vessel of the subject. This is a possibility to provide a quantification and/or a measurement of the vessel with an improved quality based on the determined quality parameter.

As mentioned above, the present invention can provide a high quality ultrasound image for measurement of the anatomical feature, since the quality parameter is determined based on the positional relation of the anatomical feature with respect to the different image planes of the two-dimensional ultrasound image data and since the image planes are correspondingly aligned. Since the alignment of the anatomical feature with respect to the image planes is performed based on the two-dimensional ultrasound image data, the alignment can be performed in real time based on a continuous data stream from the ultrasound probe so that a precise measurement with low time consumption can be provided.

### BRIEF DESCRIPTION OF THE DRAWINGS

These and other aspects of the invention will be apparent from and elucidated with reference to the embodiment(s) described hereinafter. In the following drawings
Fig. 1 shows a schematic representation of an ultrasound imaging apparatus in use to scan a volume of a patient's body;
Fig. 2a, b show ultrasound images of an anatomical feature of the patient in an image plane of a two-dimensional ultrasound image;
Fig. 3a-c show different image planes with respect to an anatomical feature for size measurements;
Fig. 4a, b show ultrasound images of an anatomical feature and indications for user guidance; and
Fig. 5 shows a schematic flow diagram of an ultrasound imaging method for inspecting an anatomical feature of a patient.

### DETAILED DESCRIPTION OF THE INVENTION

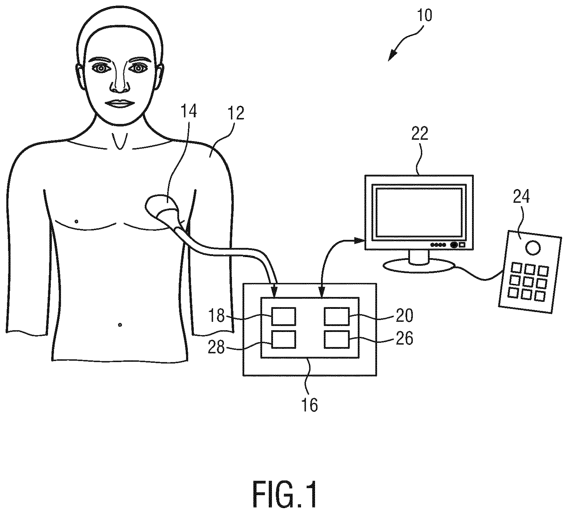

Fig. 1 shows a schematic illustration of an ultrasound imaging apparatus generally denoted by 10. The ultrasound imaging apparatus 10 is applied to inspect a volume of an anatomical site, in particular an anatomical site of a patient 12. The ultrasound imaging apparatus 10 comprises an ultrasound probe 14 having at least one transducer array including a multitude of transducer elements for transmitting and receiving ultrasound waves. The transducer elements are preferably arranged in a 2D array for providing multi-dimensional image data, in particular three-dimensional ultrasound image data and bi-plane image data. Bi-plane image data can be acquired by sweeping two intersecting 2D image planes. Generally in bi-pane imaging the two 2D planes are orthogonal to an emitting surface of the array and can intersect under a different angle.

The ultrasound imaging apparatus 10 comprises in general a control unit 16 connected to the ultrasound probe 14 for controlling the ultrasound probe 14 and for evaluating the ultrasound data received from the ultrasound probe 14.

The ultrasound probe 14 is adapted to provide three-dimensional and two-dimensional ultrasound image data in a field of view of the anatomical site of the patient 12, wherein the two-dimensional ultrasound image data is provided in two or more image planes parallel to a propagation direction of the ultrasound waves emitted by the ultrasound probe 14. The ultrasound probe 14 is in particular adapted to provide two-dimensional ultrasound images in real time in a bi-planar mode, wherein the image planes of the two-dimensional ultrasound images can be disposed (intersect) either perpendicular (orthogonal bi-planes) or under a different angle to each other. The two-dimensional ultrasound images in the two different image planes are acquired simultaneously in real-time and displayed in real-time.

The control unit 16 comprises an image processing unit 18 coupled to the ultrasound probe 14 for receiving the three-dimensional ultrasound image data and the two-dimensional ultrasound image data from the ultrasound probe 14, wherein the image processing unit 18 is adapted to determine an anatomical feature in the ultrasound image data. The image processing unit 18 determines an outline of the anatomical feature in the two-dimensional ultrasound image data based on pattern detection or edge detection and may perform a segmentation of the anatomical feature in order to provide corresponding segmentation data of the anatomical feature. In other words, the image processing unit 18 may determine main characteristics of an anatomical feature or main features of an anatomical structure.

The control unit 16 further comprises an evaluation unit 20 for evaluating the two-dimensional ultrasound image data. The evaluation unit 20 determines a positional relation of the anatomical feature with respect to the field of view of the ultrasound probe 14 and with respect to the image plane of the two-dimensional ultrasound image data and determines a quality parameter based on the positional relation of the anatomical feature in the two-dimensional ultrasound image data.

The ultrasound emitting apparatus 10 further comprises a display unit 22 for displaying image data received from the control unit 16. The display unit 22 receives the image data in general from the image processing unit 18 and is adapted to display the two-dimensional ultrasound image data and/or the three-dimensional ultrasound image data detected by the ultrasound probe 14. The ultrasound imaging apparatus 10 further comprises an input device 24 which may be connected to the display unit 22 or to the control unit 16 in order to control the image acquisition in general.

Since the ultrasound image data is utilized for measuring the anatomical feature, which is preferably a vessel of the patient 12, a precise alignment of the ultrasound probe 14 with respect to the anatomical feature and at least one of the image planes of the two-dimensional ultrasound image data is necessary. The evaluation unit 20 determines the quality parameter based on the positional relation of the anatomical feature with respect to the image plane and with respect to the viewing direction of the ultrasound probe 14 and provides a corresponding feedback to the user via the display unit 22. The evaluation unit 20 determines whether the anatomical feature is well aligned to the image planes and to the viewing direction of the ultrasound probe 14 in order to provide high quality image data so that high quality volume and/or size measurements of the anatomical feature can be achieved. The evaluation unit compares the quality parameter with predefined quality limits and provides a corresponding feedback to the user via the display unit 22.

If the quality parameter is within the predefined quality limits, a size and/or volume measurement of the anatomical feature can be performed by means of a measurement unit of the image processing unit 18 based on the two-dimensional ultrasound image data or the three-dimensional ultrasound image data received from the ultrasound probe 14 of the respective anatomical feature.

If the quality parameter is not within the predefined quality limits, an alignment unit 28 or a user guidance unit 28 of the control unit 16 indicates an alignment or an adaption of the position of the ultrasound probe 14 with respect to the anatomical feature.

In an alternative embodiment, the control unit 16 is adapted to control a steering direction of the ultrasound probe 14 based on the positional relation and the respective quality parameter received from the alignment unit 28 in order to align the image planes and/or the viewing direction of the ultrasound probe 14 electronically. The alignment may include varying an intersection angle of the image planes or their steering direction with repect to the probe (wherein the 2D planes are steered under a different than orthogonal angle with respect to the emitting surface of the array).

After the position of the ultrasound probe 14 has been aligned to the anatomical feature, the size and/or volume measurement of the anatomical feature can be performed based on the two-dimensional ultrasound image data or the three-dimensional ultrasound image data after a separate scan.

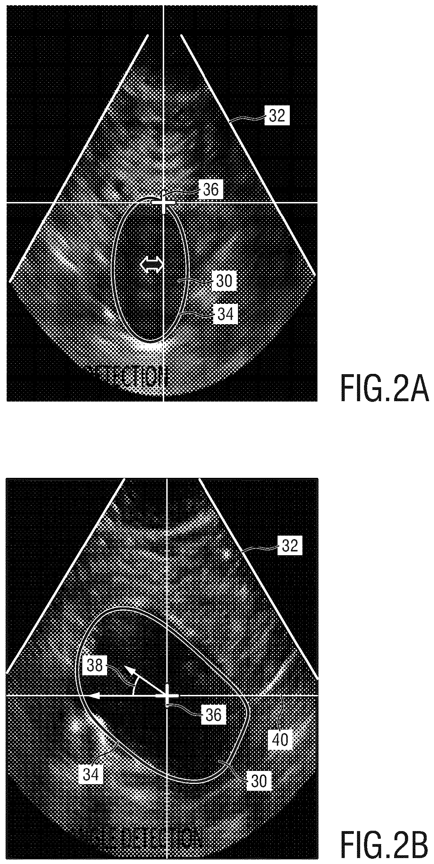

Fig. 2a, b show schematic two-dimensional ultrasound images provided by the ultrasound probe 14 and including the anatomical feature, which is generally denoted by 30. The ultrasound image data is acquired in a field of view 32 of the ultrasound probe 14.

The evaluation unit 20 determines an outline 34 of the anatomical feature 30 in order to determine the positional relation of the anatomical feature 30 with respect to the field of view 32 and/or the image plane of the two-dimensional images.

The evaluation unit 20 determines the quality parameter based on the positional relation of the anatomical feature 30 with respect to the image plane and/or the field of view 32. The positional relation on the basis on which the quality parameter is determined may be a distance of the anatomical feature 30 from an image center 36 as shown in Fig. 2a or an angle 38 of a main axis of the anatomical feature 30 with respect to a horizontal axis 40 of the two-dimensional ultrasound image as shown in Fig. 2b. In further embodiments, the quality parameter may be determined based on a depth of the two-dimensional image with respect to the anatomical feature 30 or whether the anatomical feature is entirely included in the field of view 32.

If the anatomical feature 30 is well aligned with respect to the field of view 32 and/or the image planes of the two-dimensional ultrasound images and the respective quality parameter is within the predefined quality limit, a size and/or volume measurement of the anatomical feature 30 can be performed based on the two-dimensional ultrasound image data and/or the three-dimensional ultrasound image data e.g. after a separate scan received from the ultrasound probe 14.

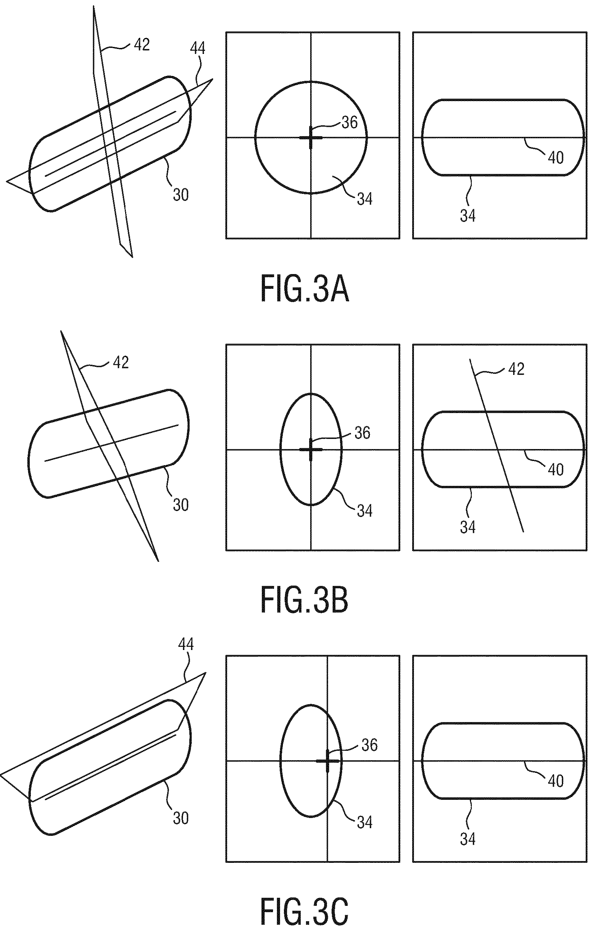

Fig. 3a-c show two intersecting image planes 42, 44 of the two-dimensional image data with respect to the anatomical feature 34 in different viewing directions and schematic sectional views of the anatomical feature 34 in the respective image planes 42, 44. The two-dimensional image data in the two image planes 42, 44 are acquired simultaneously and preferably displayed in real-time.

In Fig. 3a two image planes 42, 44 (bi-planes) are disposed with respect to the anatomical feature 30, which is a vessel of the patient 12, wherein the respective image plane 42, 44 are well aligned, i.e. the image plane 42 is disposed orthogonal to a longitudinal axis of the vessel 30 and the image plane 44 is aligned parallel to the longitudinal axis of the vessel 30 and centered with respect to the vessel 30. Consequently, the captured two-dimensional images in the image planes 42, 44 are centered with respect to the image center 36 and with respect to the horizontal axis 40 of the two-dimensional image data.

In Fig. 3b, merely the image plane 42 is shown with respect to the anatomical feature 34, wherein the image plane 42 is not disposed orthogonally to the longitudinal axis of the vessel 34 so that a misalignment of the image plane 42 is present and a correct volume and/or size measurement of the anatomical feature 30 is not possible. The respective outlines 34 of the anatomical feature 30 are schematically shown in Fig. 3b with respect to the image plane 42.

In Fig. 3c merely the image plane 44 aligned in parallel with the longitudinal axis of the anatomical feature 30 is shown, wherein the image plane 44 is not disposed in the center of the anatomical feature 30 so that the anatomical feature 30 is misaligned with respect to the center 36 and a precise measurement of the size and the volume of the anatomical feature 30 cannot be achieved as shown in Fig. 3c.

Consequently, a precise measurement of the size and/or the volume of the anatomical feature 30 which is in this case a vessel of the patient 12 can only be achieved if the two image planes 42, 44 are well-aligned with respect to the center 36 and the horizontal axis 40.

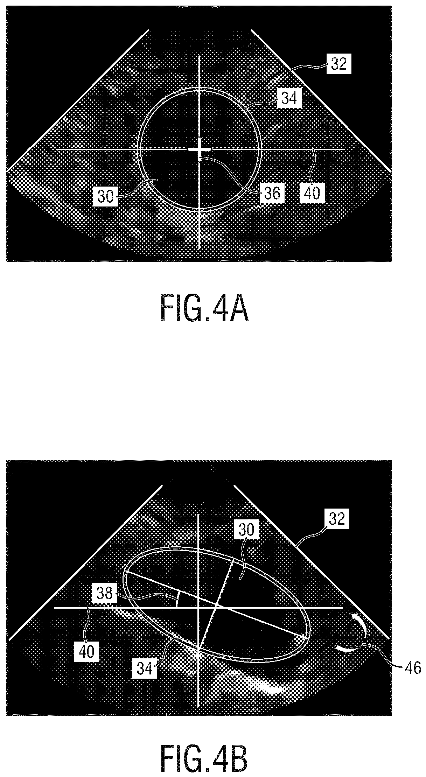

In Fig. 4 two different two-dimensional ultrasound images in the field of view 32 are schematically shown. Fig. 4a shows a two-dimensional ultrasound image including the anatomical feature 30, which is centered with respect to the center 36 of the field of view 32 and, therefore well aligned to perform a precise measurement of the size and/or the volume of the anatomical feature 30. The identified quality parameter is relatively high for this probes position with respect to the anatomical feature 30. Fig. 4b shows a two-dimensional ultrasound image including the anatomical feature 30 which is misaligned with respect to the image plane 42, 44 and the field of view 32. In this particular case, the longitudinal axis of the anatomical feature 30 is tilted with respect to the horizontal axis 40 by the angle 38. The identified quality parameter is relatively low for this probes position and planes orientation with respect to the anatomical feature 30. Measurements of the anatomical feature 30 performed using the image illustrated in Fig.4b may have an increased error with respect to a real size of the anatomical feature. The alignment unit 28 is arranged to indicate an improved alignment of the image planes with respect to the anatomical feature. In order to align the field of view 32 and the image plane 42, 44 with respect to the anatomical feature 34 an indication 46 is shown in the two-dimensional ultrasound image which indicates a rotation and/or a translation of the image planes 42, 44 or the position of the ultrasound probe 14 to align the anatomical feature 30 with respect to the field of view 32. The indication 46 (an arrow) is shown within the two-dimensional ultrasound image displayed to the user so that the user can align the image plane and/or the field of view 32 by moving the ultrasound probe 14, respectively. This invention may be beneficially implemented in vessel quantification, wherein the ultrasound planes alignment with respect to the quantified vessel may play an important role in ultrasound assisted diagnostic. Alternativly,

If the quality parameter is within predetermined quality limits a size and/or volume measurement of the anatomical feature 30 can be performed either on the basis of the two-dimensional ultrasound image data or a full three-dimensional ultrasound scan can be performed by the ultrasound probe 14 to achieve a precise size or volume measurement.

In a further embodiment, the size and/or volume measurement can be combined with an automatic segmentation of the three-dimensional ultrasound data.

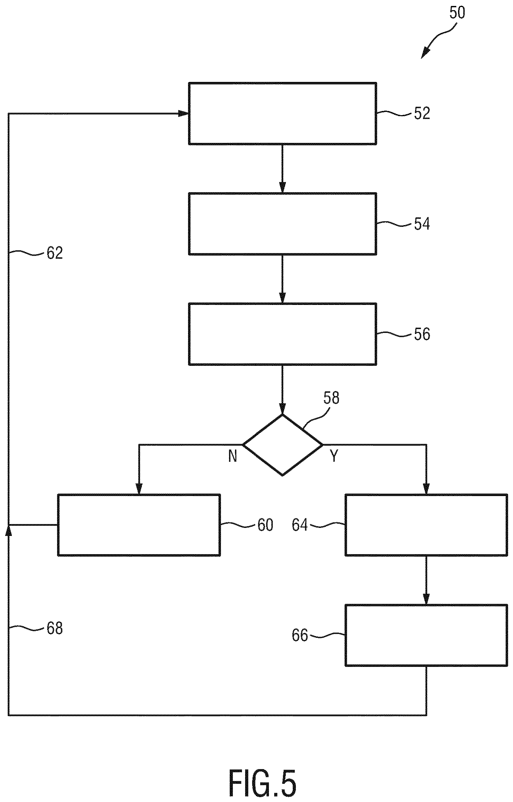

Fig. 5 shows a schematic flow diagram of an ultrasound imaging method for inspecting a volume of the subject 12. The method is in Fig. 5 generally denoted by 50.

The method 50 starts with the acquisition of the two-dimensional ultrasound image data simultaneously in the two images planes 42, 44 as shown at step 52. The anatomical feature 30 is automatically segmented in the axial and the longitudinal direction based on the two-dimensional ultrasound data in the two different image planes 42, 44 as shown at step 54. At least the orientation angle of the anatomical feature with respect to the two-dimensional image planes is estimated. This can reduce the technical effort in general, since an estimation can be simpler than a full segmentation to provide feedback for user guidance.

At step 56, the quality parameter is determined based on the positional relation of the anatomical feature 30 with respect to the respective image planes 42, 44. At step 58, the quality parameter is compared to predefined quality limits in order to evaluate the alignment quality. If the quality parameter is not within predetermined quality limit, the indication 46 is displayed on the display screen 22 as a user guidance in order to propose a rotation and/or a translation movement of the ultrasound probe 14 or an adaption of the image depth to improve the quality parameter (step 60). After the alignment, the method 50 returns to step 52 as shown by the feedback loop 62.

If the quality parameter is within the predetermined quality limit, the user gets a feedback via the display unit 22 as shown at step 64 and two-dimensional ultrasound image data or the three-dimensional ultrasound image data are acquired in the aligned position of the ultrasound probe 14 and the measurement unit may optionally determine the size and/or the volume of the anatomical feature 30 based on the two-dimensional ultrasound image data or the three-dimensional ultrasound image data as shown at step 66.

The user may optionally confirm the measurement and returns to step 52 as shown by the feedback loop 68.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. An ultrasound imaging apparatus (10) for inspecting a volume of a subject (12), comprising:
- an ultrasound probe (14) including a plurality of ultrasound transducer elements for acquiring two-dimensional ultrasound image data in two intersecting image planes in the field of view,
- an image processing unit (18) coupled to the ultrasound probe for receiving the two-dimensional ultrasound image data in the two intersecting image planes (42, 44) and for determining an anatomical feature (30) in the two intersecting image planes of the two-dimensional ultrasound image data,
- an evaluation unit (20) for evaluating the two-dimensional ultrasound image data and for determining a quality parameter based on a positional relation of the anatomical feature with respect to each of the image planes of the two-dimensional ultrasound image data in the field of view, wherein the positional relation is an outline of the anatomical feature with respect to the field of view in each of the image planes or a position of the anatomical feature with respect to an image depth of the two-dimensional ultrasound image data, and
- an alignment unit (28) for aligning or indicating an alignment of the image planes or the field of view based on the positional relation and the quality parameter, such that a position of the ultrasound probe (14) can be aligned to the anatomical feature (30),
wherein the ultrasound probe (14) is further adapted to acquire three-dimensional ultrasound data in a field of view (32) in the aligned position of the ultrasound probe (14).

2. The ultrasound imaging apparatus as claimed in claim 1, wherein the ultrasound probe is adapted to acquire the two-dimensional ultrasound image data in the two intersecting image planes in the field of view simultaneously.

3. The ultrasound imaging apparatus as claimed in claim 1, wherein the two intersecting image planes are disposed perpendicular to each other.

4. The ultrasound imaging apparatus as claimed in claim 1, further comprising a display unit (22) for displaying an ultrasound image on the basis of the two dimensional ultrasound image data, wherein the alignment unit is adapted to indicate a movement direction (46) of the ultrasound probe on the display unit based on the positional relation and the quality parameter.

5. The ultrasound imaging apparatus as claimed in claim 4, wherein the movement direction is indicated within the ultrasound image.

6. The ultrasound imaging apparatus as claimed in claim 1, further comprising a control unit (16) for controlling a steering direction of the ultrasound probe based on the positional relation and the quality parameter.

7. The ultrasound imaging apparatus as claimed in claim 1, wherein the positional relation is a distance of a center of the anatomical feature from a center (36) of the two-dimensional ultrasound image in the image plane.

8. The ultrasound imaging apparatus as claimed in claim 1, wherein the positional relation is an angle (38) between a longitudinal axis of the anatomical feature with respect to a horizontal axis of the two-dimensional ultrasound image in the image plane.

9. The ultrasound imaging apparatus as claimed in claim 1, wherein the image processing unit comprises a measurement unit for measuring a size of the anatomical feature on the basis of the two-dimensional image data.

10. The ultrasound imaging apparatus as claimed in claim 1, wherein the measurement unit comprises a segmentation unit for providing segmentation data of the two-dimensional ultrasound image data and for measuring a size of the anatomical feature on the basis of the segmentation data.

11. The ultrasound imaging apparatus as claimed in claim 1, wherein the evaluation unit comprises a segmentation unit for providing segmentation data of the anatomical feature in the two-dimensional ultrasound image data and for determining the positional relation on the basis of the segmentation data.

12. An ultrasound imaging method (50) for inspecting a volume of a subject (12), using an apparatus according to any of the previous claims, the method (50) comprising the steps of:
- acquiring (52) two-dimensional ultrasound image data in two intersecting image planes in a field of view (32) using an ultrasound probe (14),
- processing the two-dimensional ultrasound image data in the two intersecting image planes (42,44) and determining an anatomical feature (30) in the two intersecting image planes of the two-dimensional ultrasound image data,
- evaluating (56) the two-dimensional ultrasound image data and determining a quality parameter based on a positional relation of the anatomical feature with respect to the image planes of the two-dimensional ultrasound image data in the field of view, wherein the positional relation is an outline of the anatomical feature with respect to the field of view in each of the image planes or a position of the anatomical feature with respect to an image depth of the two-dimensional ultrasound image data,
- indicating (60) an alignment of the image planes or aligning (60) the image planes or the field of view based on the positional relation and the quality parameter, such that a position of the ultrasound probe (14) can be aligned to the anatomical feature (30), and
acquiring (66) three-dimensional ultrasound data in the field of view (32) in the aligned position of the ultrasound probe (14).

## Patentansprüche

1. Ultraschallbildgebungsvorrichtung (10) zum Untersuchen eines Volumens eines Subjekts (12), umfassend:
- eine Ultraschallsonde (14), die eine Vielzahl von Ultraschallwandlerelementen beinhaltet, zum Erfassen von zweidimensionalen Ultraschallbilddaten in zwei sich schneidenden Bildebenen im Sichtfeld,
- eine Bildverarbeitungseinheit (18), die mit der Ultraschallsonde gekoppelt ist, zum Empfangen der zweidimensionalen Ultraschallbilddaten in den zwei sich schneidenden Bildebenen (42, 44) und zum Bestimmen eines anatomischen Merkmals (30) in den zwei sich schneidenden Bildebenen der zweidimensionalen Ultraschallbilddaten,
- eine Auswertungseinheit (20) zum Auswerten der zweidimensionalen Ultraschallbilddaten und zum Bestimmen eines Qualitätsparameters auf Basis einer Positionsbeziehung des anatomischen Merkmals in Bezug auf jede der Bildebenen der zweidimensionalen Ultraschallbilddaten im Sichtfeld, wobei die Positionsbeziehung ein Umriss des anatomischen Merkmals in Bezug auf das Sichtfeld in jeder der Bildebenen oder eine Position des anatomischen Merkmals in Bezug auf eine Bildtiefe der zweidimensionalen Ultraschallbilddaten ist, und
- eine Ausrichtungseinheit (28) zum Ausrichten oder Angeben einer Ausrichtung der Bildebenen oder des Sichtfeldes auf Basis der Positionsbeziehung und des Qualitätsparameters derart, dass eine Position der Ultraschallsonde (14) auf das anatomische Merkmal (30) ausgerichtet werden kann,
wobei die Ultraschallsonde (14) weiter dazu geeignet ist, in der ausgerichteten Position der Ultraschallsonde (14) dreidimensionale Ultraschalldaten in einem Sichtfeld (32) zu erfassen.

2. Ultraschallbildgebungsvorrichtung nach Anspruch 1, wobei die Ultraschallsonde dazu geeignet ist, die zweidimensionalen Ultraschallbilddaten in den zwei sich schneidenden Bildebenen im Sichtfeld gleichzeitig zu erfassen.

3. Ultraschallbildgebungsvorrichtung nach Anspruch 1, wobei die zwei sich schneidenden Bildebenen senkrecht zueinander angeordnet sind.

4. Ultraschallbildgebungsvorrichtung nach Anspruch 1, die weiter eine Anzeigeeinheit (22) zum Anzeigen eines Ultraschallbildes auf Basis der zweidimensionalen Ultraschallbilddaten umfasst, wobei die Ausrichtungseinheit dazu geeignet ist, eine Bewegungsrichtung (46) der Ultraschallsonde auf der Anzeigeeinheit auf Basis der Positionsbeziehung und des Qualitätsparameters anzugeben.

5. Ultraschallbildgebungsvorrichtung nach Anspruch 4, wobei die Bewegungsrichtung innerhalb des Ultraschallbildes angegeben wird.

6. Ultraschallbildgebungsvorrichtung nach Anspruch 1, die weiter eine Steuereinheit (16) zum Steuern einer Lenkrichtung der Ultraschallsonde auf Basis der Positionsbeziehung und des Qualitätsparameters umfasst.

7. Ultraschallbildgebungsvorrichtung nach Anspruch 1, wobei die Positionsbeziehung ein Abstand eines Mittelpunkts des anatomischen Merkmals von einem Mittelpunkt (36) des zweidimensionalen Ultraschallbildes in der Bildebene ist.

8. Ultraschallbildgebungsvorrichtung nach Anspruch 1, wobei die Positionsbeziehung ein Winkel (38) zwischen einer Längsachse des anatomischen Merkmals in Bezug auf eine horizontale Achse des zweidimensionalen Ultraschallbildes in der Bildebene ist.

9. Ultraschallbildgebungsvorrichtung nach Anspruch 1, wobei die Bildverarbeitungseinheit eine Messeinheit zum Messen einer Größe des anatomischen Merkmals auf Basis der zweidimensionalen Bilddaten umfasst.

10. Ultraschallbildgebungsvorrichtung nach Anspruch 1, wobei die Messeinheit eine Segmentierungseinheit zum Bereitstellen von Segmentierungsdaten der zweidimensionalen Ultraschallbilddaten und zum Messen einer Größe des anatomischen Merkmals auf Basis der Segmentierungsdaten umfasst.

11. Ultraschallbildgebungsvorrichtung nach Anspruch 1, wobei die Auswertungseinheit eine Segmentierungseinheit zum Bereitstellen von Segmentierungsdaten des anatomischen Merkmals in den zweidimensionalen Ultraschallbilddaten und zum Bestimmen der Positionsbeziehung auf Basis der Segmentierungsdaten umfasst.

12. Ultraschallbildgebungsverfahren (50) zum Untersuchen eines Volumens eines Subjekts (12) unter Verwendung einer Einrichtung nach einem der vorstehenden Ansprüche, wobei das Verfahren (50) die folgenden Schritte umfasst:
- Erfassen (52) von zweidimensionalen Ultraschallbilddaten in zwei sich schneidenden Bildebenen in einem Sichtfeld (32) unter Verwendung einer Ultraschallsonde (14),
- Verarbeiten der zweidimensionalen Ultraschallbilddaten in den zwei sich schneidenden Bildebenen (42, 44) und Bestimmen eines anatomischen Merkmals (30) in den zwei sich schneidenden Bildebenen der zweidimensionalen Ultraschallbilddaten,
- Auswerten (56) der zweidimensionalen Ultraschallbilddaten und Bestimmen eines Qualitätsparameters auf Basis einer Positionsbeziehung des anatomischen Merkmals in Bezug auf die Bildebenen der zweidimensionalen Ultraschallbilddaten im Sichtfeld, wobei die Positionsbeziehung ein Umriss des anatomischen Merkmals in Bezug auf das Sichtfeld in jeder der Bildebenen oder eine Position des anatomischen Merkmals in Bezug auf eine Bildtiefe der zweidimensionalen Ultraschallbilddaten ist,
- Angeben (60) einer Ausrichtung der Bildebenen oder Ausrichten (60) der Bildebenen oder des Sichtfeldes auf Basis der Positionsbeziehung und des Qualitätsparameters derart, dass eine Position der Ultraschallsonde (14) auf das anatomische Merkmal (30) ausgerichtet werden kann, und
Erfassen (66) von dreidimensionalen Ultraschalldaten im Sichtfeld (32) in der ausgerichteten Position der Ultraschallsonde (14).

## Revendications

1. Appareil d'échographie (10) pour inspecter un volume d'un sujet (12), comprenant :
- une sonde échographique (14) incluant une pluralité d'éléments transducteurs à ultrasons pour acquérir des données d'image échographique bidimensionnelle dans deux plans d'image sécants dans le champ de vision,
- une unité de traitement d'image (18) couplée à la sonde échographique pour recevoir les données d'image échographique bidimensionnelle dans les deux plans d'image sécants (42, 44) et pour déterminer une caractéristique anatomique (30) dans les deux plans d'image sécants des données d'image échographique bidimensionnelle,
- une unité d'évaluation (20) pour évaluer les données d'image échographique bidimensionnelle et pour déterminer un paramètre de qualité sur la base d'une relation de position de la caractéristique anatomique par rapport à chacun des plans d'image des données d'image échographique bidimensionnelle dans le champ de vision, dans lequel la relation de position est un contour de la caractéristique anatomique par rapport au champ de vision dans chacun des plans d'image ou une position de la caractéristique anatomique par rapport à une profondeur d'image des données d'image échographique bidimensionnelle, et
- une unité d'alignement (28) pour aligner ou indiquer un alignement des plans d'image ou du champ de vision sur la base de la relation de position et du paramètre de qualité, de telle sorte qu'une position de la sonde échographique (14) puisse être alignée sur la caractéristique anatomique (30),
dans lequel la sonde échographique (14) est en outre adaptée pour acquérir des données échographiques tridimensionnelles dans un champ de vision (32) dans la position alignée de la sonde échographique (14).

2. Appareil d'échographie selon la revendication 1, dans lequel la sonde échographique est conçue pour acquérir simultanément les données d'image échographique bidimensionnelle dans les deux plans d'image sécants dans le champ de vision.

3. Appareil d'échographie selon la revendication 1, dans lequel les deux plans d'image sécants sont disposés perpendiculairement l'un à l'autre.

4. Appareil d'échographie selon la revendication 1, comprenant en outre une unité d'affichage (22) pour afficher une image échographique sur la base des données d'image échographique bidimensionnelle, dans lequel l'unité d'alignement est conçue pour indiquer un sens de déplacement (46) de la sonde échographique sur l'unité d'affichage sur la base de la relation de position et du paramètre de qualité.

5. Appareil d'échographie selon la revendication 4, dans lequel le sens de déplacement est indiqué dans l'image échographique.

6. Appareil d'échographie selon la revendication 1, comprenant en outre une unité de commande (16) pour commander une direction d'orientation de la sonde échographique sur la base de la relation de position et du paramètre de qualité.

7. Appareil d'échographie selon la revendication 1, dans lequel la relation de position est une distance d'un centre de la caractéristique anatomique à partir d'un centre (36) de l'image échographique bidimensionnelle dans le plan d'image.

8. Appareil d'échographie selon la revendication 1, dans lequel la relation de position est un angle (38) entre un axe longitudinal de la caractéristique anatomique par rapport à un axe horizontal de l'image échographique bidimensionnelle dans le plan d'image.

9. Appareil d'échographie selon la revendication 1, dans lequel l'unité de traitement d'image comprend une unité de mesure pour mesurer une taille de la caractéristique anatomique sur la base des données d'image bidimensionnelle.

10. Appareil d'échographie selon la revendication 1, dans lequel l'unité de mesure comprend une unité de segmentation pour fournir des données de segmentation des données d'image échographique bidimensionnelle et pour mesurer une taille de la caractéristique anatomique sur la base des données de segmentation.

11. Appareil d'échographie selon la revendication 1, dans lequel l'unité d'évaluation comprend une unité de segmentation pour fournir des données de segmentation de la caractéristique anatomique dans les données d'image échographique bidimensionnelle et pour déterminer la relation de position sur la base des données de segmentation.

12. Procédé d'échographie (50) pour inspecter un volume d'un sujet (12), en utilisant un appareil selon l'une quelconque des revendications précédentes, le procédé (50) comprenant les étapes consistant à :
- acquérir (52) des données d'image échographique bidimensionnelle dans deux plans d'image sécants dans un champ de vision (32) à l'aide d'une sonde échographique (14),
- traiter les données d'image échographique bidimensionnelle dans les deux plans d'image sécants (42, 44) et déterminer une caractéristique anatomique (30) dans les deux plans d'image sécants des données d'image échographique bidimensionnelle,
- évaluer (56) les données d'image échographique bidimensionnelle et déterminer un paramètre de qualité sur la base d'une relation de position de la caractéristique anatomique par rapport aux plans d'image des données d'image échographique bidimensionnelle dans le champ de vision, dans lequel la relation de position est un contour de la caractéristique anatomique par rapport au champ de vision dans chacun des plans d'image ou une position de la caractéristique anatomique par rapport à une profondeur d'image des données d'image échographique bidimensionnelle,
- indiquer (60) un alignement des plans d'image ou aligner (60) les plans d'image ou le champ de vision sur la base de la relation de position et du paramètre de qualité, de telle sorte qu'une position de la sonde échographique (14) puisse être alignée sur la caractéristique anatomique (30), et
acquérir (66) des données échographiques tridimensionnelles dans le champ de vision (32) dans la position alignée de la sonde échographique (14).
